# EUROPEAN PATENT SPECIFICATION

(11) **EP 0 783 328 B1**
(45) Date of publication and mention of the grant of the patent: **06.11.2002**
(21) Application number: 95922026.0
(22) Date of filing: 12.05.1995
(51) Int. Cl.: A61L 27/00, C23C 8/24, A61F 2/24

(54) **METHOD OF IMPROVING MECHANICAL HEART VALVE PROSTHESES AND OTHER MEDICAL INSERTS, AND PRODUCTS MANUFACTURED IN ACCORDANCE WITH THE METHOD**
VERFAHREN ZUR VERBESSERUNG MECHANISCHER HERZKLAPPENPROTHESE UND ANDERER MEDIZINISCHER IMPLANTATE UND NACH DIESEM VERFAHREN HERGESTELLTE PRODUKTE
PROCEDE D'AMELIORATION DE VALVULES PROTHETIQUES MECANIQUES ET AUTRES IMPLANTS MEDICAUX, ET PRODUITS FABRIQUES SELON CE PROCEDE

(30) Priority: 08.06.1994 SE 9401967
(43) Date of publication of application: 16.07.1997
(73) Proprietor: Olin, Christian, 582 52 Linköping (SE)
(72) Inventor: OLIN, Christian, S-582 52 Linköping (SE); JOHANSSON, Erik, S-756 43 Uppsala (SE)
(74) Representative: Lautmann, Kurt O.
(86) International application number: SE9500530
(87) International publication number: WO95033501

(56) References cited:
- EP-A- 0 449 793
- WO-A-92/02197

## Description

The invention relates to a method of improving mechanical heart valve prostheses and other components implanted in a circulatory system in the human body, by reducing their tendency to contribute to forming thrombi and also by improving the mechanical and biocompatible properties of the products. The invention also relates to products manufactured using the method.

### Background

In most patients undergoing surgical treatment of acquired heart valve disease, the valve is so damaged that it cannot be repaired but has to be replaced with a prosthetic valve. Currently available prosthetic heart valves are of two general types: biological and mechanical.

Biological valves are usually made of biological tissue, treated with glutaraldehyde, and mounted on stents. The main advantage of biological valves is that they can frequently be used without anticoagulants. The disadvantage is that they have a tendency to calcify in time, particularly in the case of younger patients.

Mechanical valves are usually made of pyrolytic carbon or combinations of pyrolytic carbon and metal. The advantage of mechanical valves is their long-term durability, the disadvantage is the need for lifelong anticoagulant therapy and the disturbing noise they sometimes produce.

The two most commonly used mechanical heart valves today are tilting disc valves and bileaflet valves. In recent years bileaflet valves have gained in popularity and have largely replaced the tilting, disc valves. The reason is their relative ease of implantation, particularly in the mitral position, together with a low noise level and relatively low incidence of thromboembolic complications.

The most commonly used bileaflet heart valve prosthesis today is the St. Jude valve. It was clinically introduced in 1977 and has now been used in more than 500,000 patients. The valve is made completely of pyrolytic carbon and consists of a valve house with two flat, semicircular leaflets, suspended in a hinge mechanism that permits the leaflet to open to 85 degrees.

Another bileaflet valve which has gained in popularity in recent years is the Carbomedics heart valve. It is superficially similar to the St. Jude valve, but has a slightly different hinge mechanism and opens to 78 degrees. It has a metal (titanium) ring around the valve house in order to increase its stiffness, thereby reducing the risk of valve leakage and improving the X-ray visibility of the valve.

A third bileaflet valve which has recently been introduced on the market is the Sorin Bicarbon valve. In contrast to the two bileaflet valves mentioned above, the Bicarbon valve is manufactured in Europe. It has a valve house made of titanium coated with a thin film of turbostratic carbon (Carbofilm) to improve hemocompatibility. The use of titanium in the valve house facilitates manufacture and improves X-ray visibility.

### Drawbacks of the currently available mechanical heart valves

Although most of the currently used mechanical heart valves today perform well, they have a number of drawbacks. Among them may be mentioned:
1. Due to imperfections in design, there is a continuous destruction of blood elements (platelets, white and red blood cells) resulting in activation of the patient's coagulation system and thus a tendency for thrombi to form in the valve. To prevent thromboembolic complications the patient has to undergo lifelong anticoagulant therapy which in itself, if not properly monitored, can lead to life-threatening bleeding.
2. In patients with impaired heart function and high pulse rate, the leakage through the valve at closure can be high. This is particularly true for large size prostheses in the mitral position where the leakage may be as high as 30% of the stroke volume.
3. In young and physically active patients the sound of the prosthesis may be disturbing to the patient and his surroundings.
4. The valves are expensive to manufacture, the current cost being about SEK 20,000 per piece.

### Desired characteristics of an ideal mechanical heart valve

1) It should have good hemodynamic performance, i.e. permit unobstructed forward flow and close with little regurgitation.
2) It should have a physiological flow pattern with absence of shear stress and regurgitant jets.
3) It should cause as little activation as possible of the patient's coagulation system, and thus be resistant to thromboembolism, even if no conventional anticoagulants are used.
4) It should be easy and safe to implant, even by inexperienced surgeons. The moving parts should be protected by the valve house and preferably move in the same direction as the natural valve leaflet, permitting the prosthesis to be implanted in the mitral ostium without having to remove the mitral valve apparatus (important for the left ventricular function).
5) It should be mechanically reliable and be resistant to wear.
6) It should be possible to monitor the valve function noninvasively, i.e. by echocardiography or by X-ray.
7) The sound produced by the prosthesis should not be disturbing to the patient.
8) It should be easy to manufacture and market.

A new design for mechanical heart valve prosthesis is shown in Figures 1-10. (C. Olin, Swedish patent application No. 9002603-0). This is a bileaflet design but according to a new principle. The leaflet moves in the same direction as the natural leaflet and has a shorter range of movement than other bileaflet designs. The leaflets are gently curved in the transverse and longitudinal directions, thereby improving blood flow. Faster and better closing is also obtained.

### Material properties of heart valve prosthesis according to the invention.

Extremely high demands are placed on material, surface and design in order to fulfil the above requirements for an ideal mechanical heart valve while avoiding the drawbacks described, and complying with the desired manufacturing aspects.

Titanium and titanium alloys are well known materials for use in prostheses, implants and other biotechnical applications, thanks to their unique properties with regard to corrosion and biocompatibility, and to the combination of low density and high mechanical strength. The use of titanium and titanium alloys in applications with sliding contact always causes serious wear and friction problems since relatively low hardness and properties relating to adhesion and oxidation result is serious problems with friction and wear. An unpredictable increase in friction and wear often leads to unacceptable functional disturbances.

According to the present invention many of the problems regarding mechanical heart valve prosthes described above, can be reduced or completely eliminated by using a specially developed surface-treating method for titanium and titanium alloys, the TiSurf Prosess® . This method creates a purely ceramic surface layer consisting of titanium nitride (TiN). The surface layer is hard, gives low friction and negligible wear. Results, not yet published, from experiments testing the tendency to form thrombi show entirely unique and improved properties for titanium and titanium alloys where the surface has been transformed by use of this method, in comparison with conventional materials used in heart valve prostheses.

According to the above-mentioned new treating method, TiSurf Prosess® , titanium Or titanium alloys are treated with nitrogen gas under vacuum at a temperature of 650 - 1000°C so that the surface layer of the metal is transformed to a completely uniformly distributed nitride layer having uniform thickness and high purity. The surface is also coated internally and in places difficult to access, such as inside tubes, capillaries, etc. This nitride layer has high hardness, approximately 2000 HV 0.05, and exceptionally good adhesion. The layer has good friction and wear properties, anticorrosion properties and good biocompatible properties. If titanium alloys are used as starting material a pure nitride layer is obtained which is impoverished of damaging alloy metals.

According to the TiSurf process (Erik Johansson, Helena Westberg, European patent application No. 91850067-9) a nitride layer is obtained that has greatly improved properties in comparison with previously known methods. The method is furthermore simple and inexpensive to perform. The invention relates to a method of improving the properties of mechanical heart valve prostheses both with regard to their tendency to contribute to the formation of thrombi and with regard to mechanical and biocompatible properties, by coating the surface with a nitride layer in accordance with the TiSurf process. The method can also be used on other mechanical products to be implanted in the body in contact with the circulating blood, such as blood pumps. The invention also applies to products manufactured by means of the method.

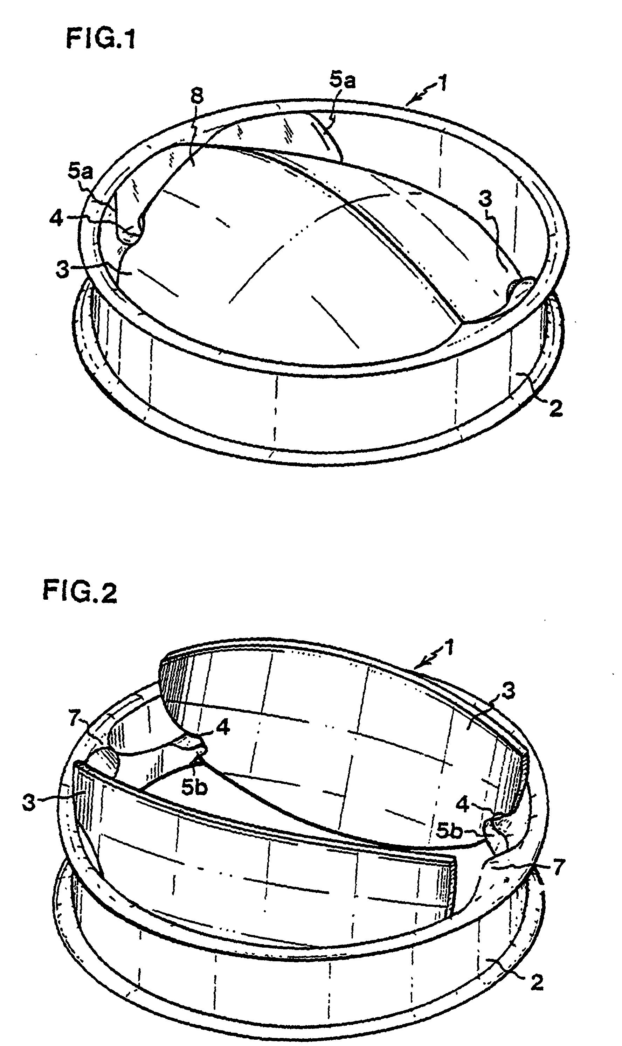

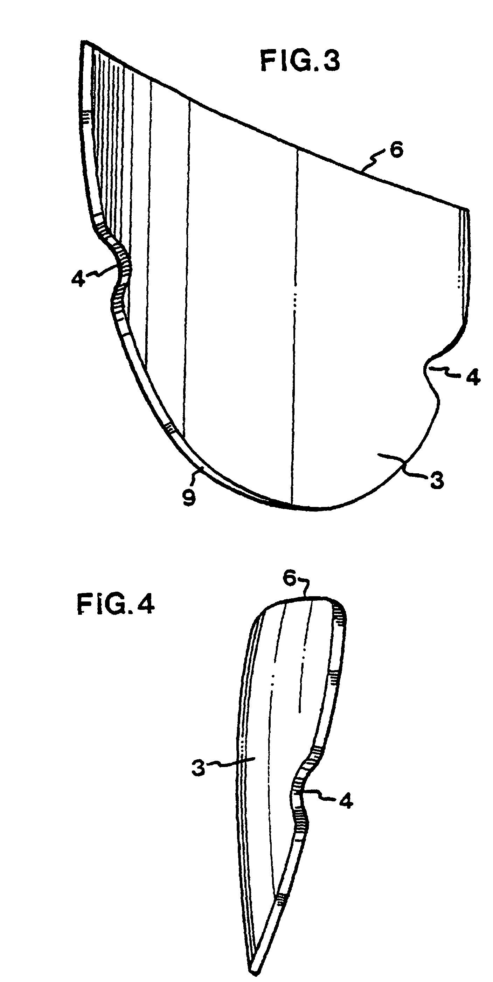

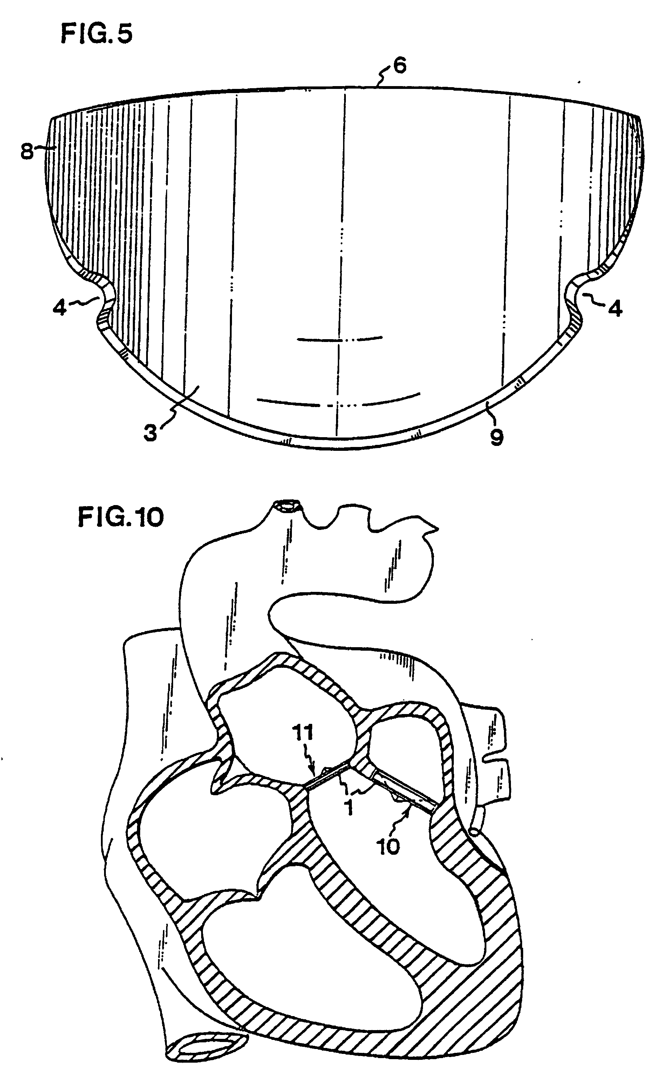

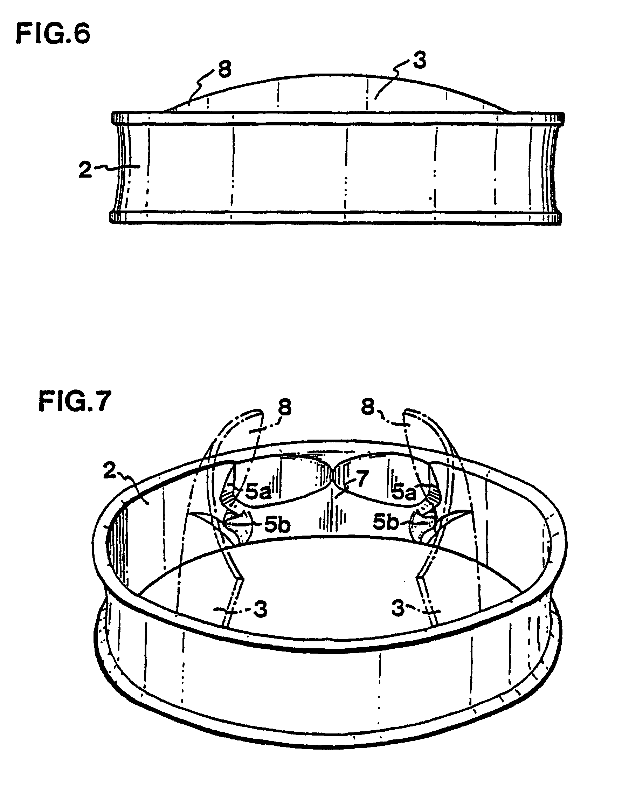

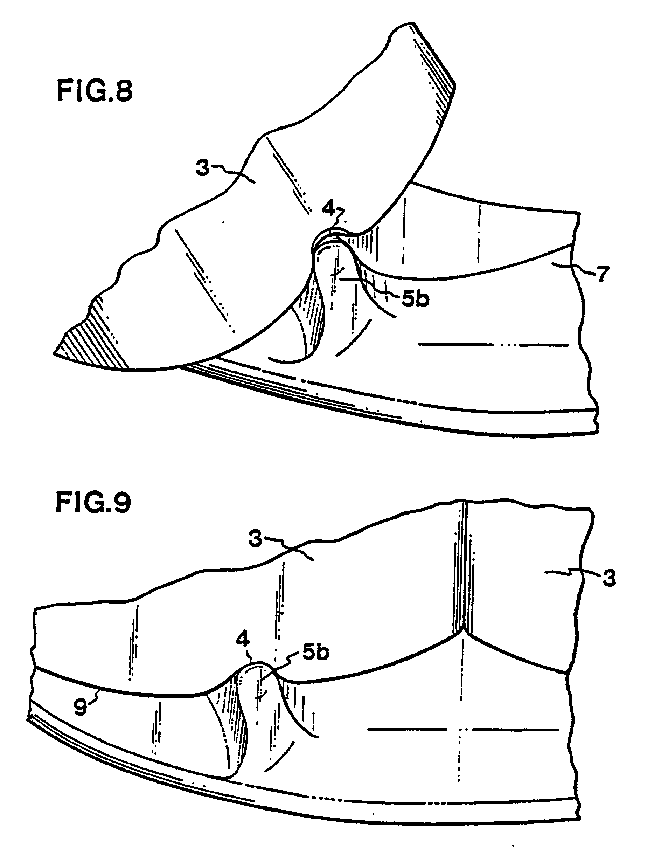

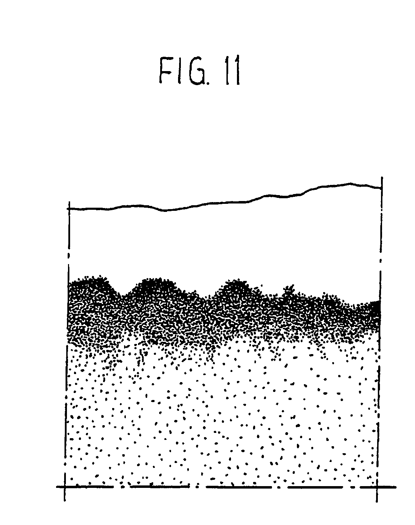

More specifically the invention relates to a mechanical bileaflet heart valve made of titanium or titanium alloy with a nitride layer in the surface, as described above. A heart valve in accordance with the above-mentioned design by C. Olin for which a patent is pending (Swedish patent application No. 9002603-0) is shown by way of example. The valve was made of titanium in accordance with the invention, and surface-treated in accordance with the invention to produce a surface layer of nitride. The heart valve has a unique design as shown in Figures 1-10. The combination of material properties in the surface-treated titanium and the design of the heart valve provides all the desired advantages of an ideal heart valve. The surface-treated titanium has proved to have less tendency to cause thrombi upon implantation in the circulatory system than pyrolytic carbon, for instance, which is currently the material predominantly used. Thanks to the above-mentioned surface treatment the entire heart valve can be made of titanium, which greatly reduces the cost of manufacture, as well as facilitating checking the function by X-ray. The combination of a unique design of the heart valve and unique material properties enabling use without anticoagulants and thus making subsequent control unnecessary may offer opportunities of world-wide use of this new heart valve. The unique material can also be used for other components to be implanted in the human body, and give products with less tendency to contribute to the formation of thrombi and with improved mechanical and biocompatible properties.

### Brief description of the drawings

- Figures 1-10: show a heart valve prosthesis according to Swedish patent application No. 9002603-0 made of titanium with a surface layer in accordance with the invention,
- Figure 11: shows the surface, seen in cross section, of a component made of the titanium alloy Ti-6Al-4V (ASTM Grade 5) with a pure, coherent nitride layer in the surface according to the TiSurf method (TiSurf Process® . The surface layer has light contrast and the picture was taken using a scanning electron microscope (SEM).
- Figures 12-13: show surfaces of various materials implanted in the circulatory system of laboratory animals and
- Figure 14: shows a diagram over the thrombi deposits on the material examined.

### Example 1

Experiment showing reduced risk of thrombi.

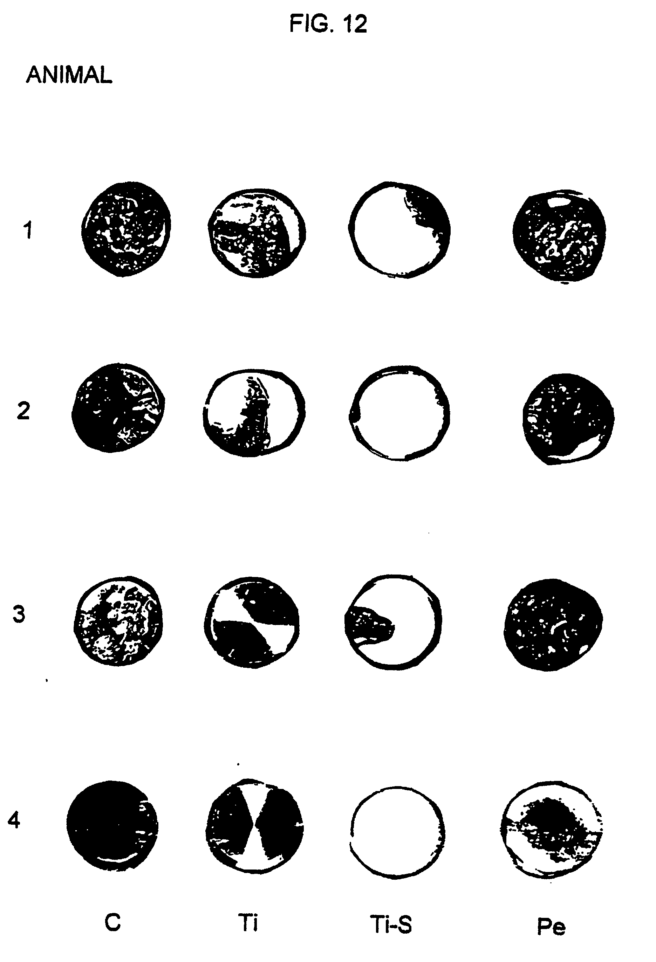

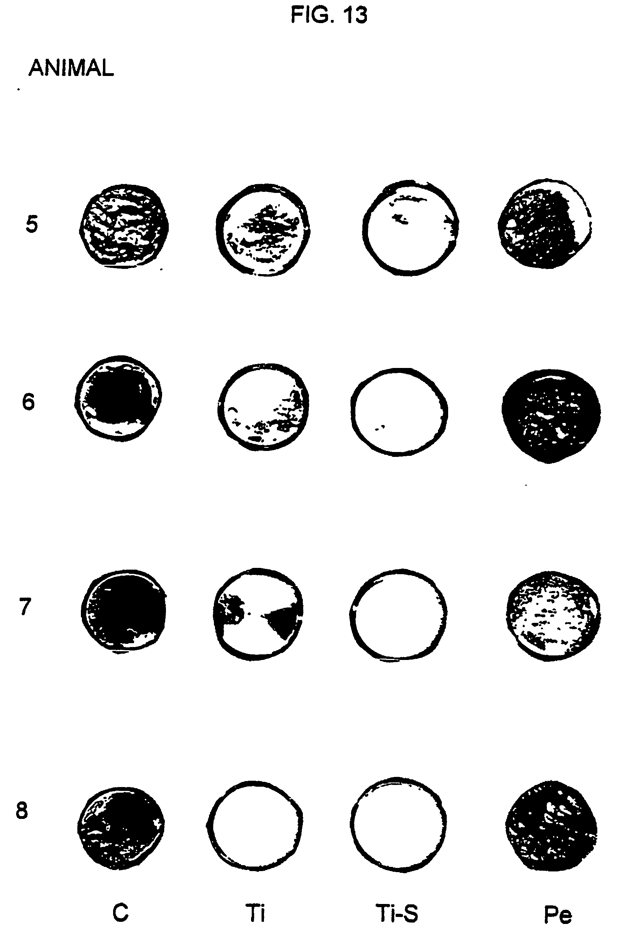

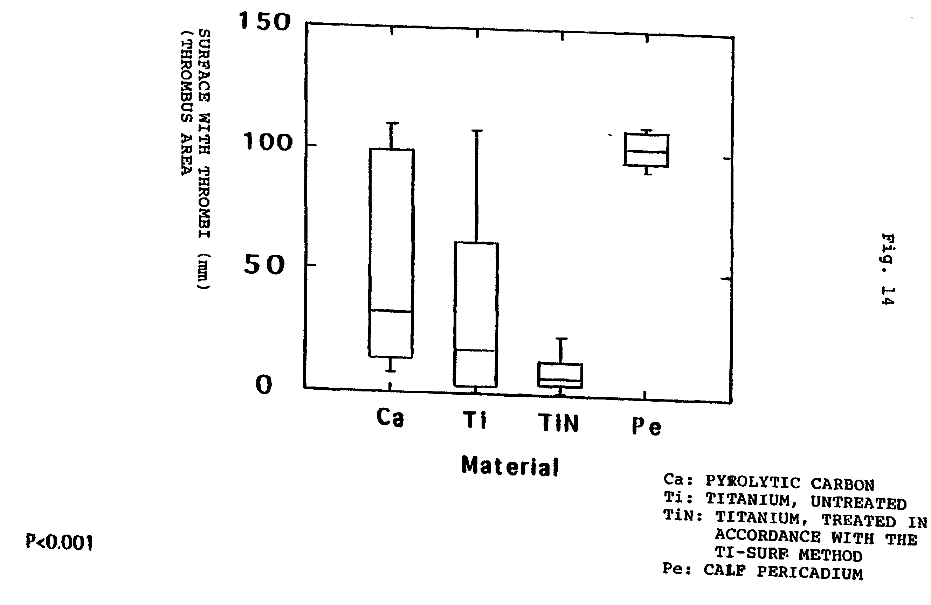

Experiments were performed on sheep in order to study the surface of titanium and titanium alloy (Ti-6Al-4V) treated by means of the TiSurf Process® , with regard to the tendency of the Ti surface to give rise to the formation of thrombi and to compare its properties in this respect with material currently used for heart valve prostheses. Discs with the same surface finish as in valve prostheses were implanted in the circulatory systems of anaesthetised sheep via a section in the thorax. Up to four different materials could be studied and compared simultaneously in each sheep, and eight sheep were used in all (Fig. 12-13). The materials studied were pyrolytic carbon (Pyrolite® ) (C), titanium Gr5 (Ti-6Al-4V), (Ti), Titanium Gr 5 surface-treated with the TiSurf Process® T1-6Al-4V (Ti-S) and the pericardium of calf treated with glutaraldehyde (Pe). The latter material was taken from leaflets in biological valve prostheses and wrapped around the plastic buttons of Delrin used to place the discs in contact with the blood flow. After two hours the discs were removed and photographed beside each other (Fig. 12-13). They were then fixed in buffered glutaraldehyde solution and photographed again one by one, enlarged approximately three times. The "thrombus area" was then calculated from these photographs, using planimetric technique, in square millimetres on each disc on which there were thrombi. In assessing, it should be observed that two of the Ti-S discs had thrombi which probably came from adjacent vessel walls that had been damaged and which should not really have been counted (but were included any way). The results (not yet published) show that the Ti-Surf surface had considerably less thrombi deposits (three-star significance) than the other materials (See diagram, Fig. 14).

## Claims

1. A method of improving the properties of prostheses and other mechanical parts for implantation in the human body in a circulatory system, by reducing their tendency to contribute to the formation of thrombi and by improving their mechanical and biocompatible properties,
**characterized in that** said prostheses made of titanium or titanium alloys are treated with nitrogen gas under vacuum at a temperature of 650 - 1000 ° C so that the surface layer of the products is transformed to a completely uniformly distributed nitride layer having uniform thickness and high purity.

2. Prostheses or other mechanical parts for implantation in a human body in a circulatory system,
**characterized in that** they are made of titanium or titanium alloy with a ceramic surface layer of titanium nitride obtainable by the method of claim 1

3. Products according to claim 2,
**characterized in that** they are made of a titanium alloy.

4. A product according to claim 2, which is mechanical heart valve.

5. A product according to claim 2, which is heart valve prosthesis.

6. A product according to claim 2, which is a blood pump.

## Patentansprüche

1. Verfahren zum Verbessern der Eigenschaften von Prothesen und anderen mechanischen Teilen für die Implantation in ein Zirkulationssystem im menschlichen Körper durch ein Verringern ihrer Neigung, zur Bildung von Thromben beizutragen, und durch ein Verbessern ihrer mechanischen und biokompatiblen Eigenschaften,
**dadurch gekennzeichnet, daß** diese aus Titan oder Titanlegierungen hergestellten Prothesen unter Vakuum bei einer Temperatur von 650 bis 1000°C mit Stickstoffgas derart behandelt werden, daß die Oberflächenschicht der Produkte in eine vollständig gleichförmig verteilte Nitrid-Schicht mit gleichmäßiger Dicke und hoher Reinheit umgewandelt wird.

2. Prothesen oder andere mechanische Teile für die Implantation in ein Zirkulationssystem in einem menschlichen Körper,
**dadurch gekennzeichnet, daß** sie aus Titan oder Titanlegierung mit einer keramischen Oberflächenschicht aus Titannitrid hergestellt sind, die durch das Verfahren gemäß Anspruch 1 erhältlich ist.

3. Gegenstände nach Anspruch 2,
**dadurch gekennzeichnet, daß** sie aus Titanlegierung hergestellt sind.

4. Gegenstand nach Anspruch 2, bei dem es sich um eine mechanische Herzklappe handelt.

5. Gegenstand nach Anspruch 2, bei dem es sich um eine Herzklappenprothese handelt.

6. Gegenstand nach Anspruch 2, bei dem es sich um eine Blutpumpe handelt.

## Revendications

1. Méthode pour améliorer les propriétés de prothèses et d'autres pièces mécaniques pour implantation dans le corps humain dans un système circulatoire, en réduisant leur tendance à contribuer à la formation de thromboses et en améliorant leurs propriétés mécaniques et biocompatibles,
**caractérisée en ce que** lesdites prothèses faites de titane ou d'alliages de titane sont traitées au gaz nitrogène sous vide à une température se situant entre 650 et 1000°C de manière que la couche surfacique des produits soit transformée en une couche de nitrure complètement uniformément répartie, ayant une épaisseur uniforme et une haute pureté.

2. Prothèses ou autres pièces mécaniques pour implantation dans un corps humain dans un système circulatoire,
**caractérisées en ce qu'**elles sont faites de titane ou d'alliage de titane avec une couche surfacique céramique en nitrure de titane obtenable par la méthode selon la revendication 1.

3. Produits selon la revendication 2,
**caractérisés en ce qu'**ils sont faits d'un alliage de titane.

4. Produit selon la revendication 2, qui est une valve cardiaque mécanique.

5. Produit selon la revendication 2, qui est une prothèse valvulaire cardiaque.

6. Produit selon la revendication 2, qui est une pompe à sang.
